# EUROPEAN PATENT SPECIFICATION

(11) **EP 2 048 224 B1**
(45) Date of publication and mention of the grant of the patent: **06.06.2012**
(21) Application number: 06781420.2
(22) Date of filing: 21.07.2006
(51) Int. Cl.: C12N 9/00, C12N 9/88, C12P 7/62

(54) **MICROORGANISM WITH REPLACED GENE AND PROCESS FOR PRODUCING POLYESTER USING THE SAME**
MIKROORGANISMUS MIT AUSGETAUSCHTEM GEN UND VERFAHREN ZUR HERSTELLUNG VON POLYESTER DAMIT
MICRO-ORGANISME DOTÉ D'UN GÈNE REMPLACÉ ET PROCÉDÉ DE PRODUCTION DE POLYESTER À L'AIDE DUDIT MICRO-ORGANISME

(43) Date of publication of application: 15.04.2009
(73) Proprietor: Kaneka Corporation, Osaka-shi, Osaka 530-8288 (JP); Meredian, Inc., Bainbridge, GA 39819 (US)
(72) Inventor: MARUYAMA, Hiroyuki, Kakogawa-shi, Hyogo 675-0051 (JP)
(74) Representative: Isarpatent
(86) International application number: PCT/JP2006/314498
(87) International publication number: WO 2008/010296

(56) References cited:
- EP-A- 0 824 148
- WO-A-99/14313
- WO-A1-03/050277
- WO-A1-2004/074476
- WO-A1-2005/098001
- JP-A- 10 108 682
- JP-A- 2005 333 933
- US-B1- 6 593 116
- TSUGE T ET AL: "Biosynthesis and compositional regulation of poly[(3-hydroxybutyrate)-co-(3-hydroxyhexa noate)] in recombinant Ralstonia eutropha expressing mutated polyhydroxyalkanoate synthase genes" MACROMOLECULAR BIOSCIENCE, WILEY VCH VERLAG, WEINHEIM, DE, vol. 4, no. 3, 15 March 2004 (2004-03-15), pages 238-242, XP003011958 ISSN: 1616-5187
- FUKUI T ET AL: "Efficient production of polyhydroxyalkanoates from plant oils by alcaligenes eutrophus and its recombinant strain" APPLIED MICROBIOLOGY AND BIOTECHNOLOGY, SPRINGER VERLAG, BERLIN, DE, vol. 49, no. 3, 1 March 1998 (1998-03-01), pages 333-336, XP002979648 ISSN: 0175-7598
- KAHAR P ET AL: "High yield production of polyhydroxyalkanoates from soybean oil by Ralstonia eutropha and its recombinant strain" POLYMER DEGRADATION AND STABILITY, BARKING, GB, vol. 83, no. 1, 1 January 2004 (2004-01-01), pages 79-86, XP004496300 ISSN: 0141-3910
- YORK GREGORY M ET AL: "Accumulation of the PhaP phasin of Ralstonia eutropha is dependent on production of polyhydroxybutyrate in cells" JOURNAL OF BACTERIOLOGY, vol. 183, no. 14, July 2001 (2001-07), pages 4217-4226, XP002553353 ISSN: 0021-9193
- KICHISE T. ET AL.: 'Enhanced accumulation and changed monomer composition in polyhydroxyalkanoate (PHA) copolyester by in vitro evolution of Aeromonas caviae PHA synthase' APPL. ENVIRON. MICROBIOL. vol. 68, no. 5, 2002, pages 2411 - 2419, XP002961039

## Description

### TECHNICAL FIELD

The present invention relates to an improved microbial strain useful for a commercial production of a polyhydroxyalkanoic acid (PHA), which is a biodegradable polyester, and a production method of PHA using the same.

### BACKGROUND ART

Polyhydroxyalkanoic acids are polyester-type organic polymers produced by a wide range of microorganisms. These polymers are biodegradable, are thermoplastic polymers, and are producible from recyclable resources, so that attempts for an industrial production of the polymers as environment-conscious materials or biocompatible materials to be used for various industries have been conducted.

The monomer constituting said polyester is 3-hydroxyalkanoic acid in the general name. Specifically, its polymer molecule is formed by homopolymerization or copolymerization of 3-hydroxybutyric acid, 3-hydroxyvaleric acid, 3-hydroxyhexanoic acid, 3-hydroxyoctanoic acid, or 3-hydroxyalkanoic acid having a longer alkyl chain. Poly-3-hydroxybutyric acid (hereinafter abbreviated as "P(3HB)"), which is a homopolymer of 3-hydroxybutyric acid (hereinafter abbreviated as "3HB"), was first discovered in Bacillus megaterium in 1925. However, since P(3HB) is high in crystallinity, it is hard and brittle, so that the range of practical application thereof is limited. Therefore, studies have been undertaken to improve these properties.

TSUGE T ET AL: MICROMOLECULAR BIOSCIENCE, vol. 4, no. 3, 15 March 2004, pages 238-242, XP003011958 describes the introduction of the PHA synthase gene from A. caviae into R. eutropha PHB 4 (DSM541) which is derived from R. eutropha H16 and lacks the polyester synthase. The so obtained R. eutropha strains produced a copolyester of 3HB and 3HH. The introduction of the PHA synthase gene from A. caviae into R. eutropha was performed via plasmids.

Among others, a process for producing a copolymer made of 3-hydroxybutyric acid (3HB) and 3-hydroxyvaleric acid (3HV) (hereinafter abbreviated as P(3HB-co-3HV)) is disclosed (for example, refer to Patent Document 1 and Patent Document 2). This P(3HB-co-3HV) is rich in flexibility as compared with P(3HB), hence was expected to have a broader application range. In actuality, however, P(3HB-co-3HV) shows only slight changes in physical properties even when the molar fraction of 3HV is increased. In particular, the flexibility can not be improved. Thus, it has been used only in the field of rigid shaped articles such as shampoo bottles and disposable razor grips.

Moreover, a medium-chain PHA constituted of 3-hydroxyalkanoic acids having 6 to 16 carbon atoms in the alkyl chain is known to be in lower crystallinity as compared with P(3HB) or P(3HB-co-3HV), and rich in elasticity (refer to Non-Patent Document 1). Thus, applications for different fields are hoped for. Studies for producing the medium-chain PHA has been conducted by introducing a PHA synthase gene of the genus Pseudomonas into the genus Pseudomonas, the genus Ralstonia, or Escherichia coli, but all of these processes were not suitable for the industrial production due to low productivity (refer to Non-Patent Document 2; Non-Patent Document 3; and Non-Patent Document 4).

In recent years, studies have been made concerning the copolyester consisting of two components 3HB and 3-hydroxyhexanoic acid (hereinafter abbreviated as 3HH) (hereinafter abbreviated as P(3HB-co-3HH)) and the process for producing it (refer to Patent Document 3 and Patent Document 4). According to these patent documents, this process for producing P(3HB-co-3HH) comprises fermentative production thereof from fatty acids, such as oleic acid, or oils and fats, such as olive oil, using Aeromonas caviae isolated from soil. Studies concerning the properties of P(3HB-co-3HH) have also been made (refer to Non-Patent Document 5). According to this report, when Aeromonas caviae is cultured using fatty acids of not less than 12 carbon atoms as the only carbon source, P(3HB-co-3HH) with a 3HH composition of 11 to 19 mol% can be fermentatively produced. It has been revealed that the properties of such P(3HB-co-3HH) change from hard and brittle gradually to soft and flexible, to an extent exceeding the flexibility of P(3HB-co-3HV), with the increase in the 3HH composition. That is, P(3HB-co-3HH) can be given a wide applicable range of physical properties, from properties of rigid polyesters to properties of flexible polyesters, by changing the 3HH composition and therefore can be expected to be applicable in a wide range, from a chassis for a TV-set and the like, for which rigidity is required, to films and the like, for which flexibility is required. However, with this production process, the productivity of polyester was as low as the cell production of 4 g/L and polyester content of 30%, and the process has yet to be said insufficient as the process for the practical use of the polyester. Therefore, a process providing higher productivity has been searched for toward the practical use.

Attempts aiming at the industrial production of P(3HB-co-3HH) have also been conducted. Among cultures using Aeromonas hydrophila, in a 43-hour feed culture using oleic acid as a carbon source, P(3HB-co-3HH) with the cell productivity of 95.7 g/L, polyester content of 45.2% and 3HH composition of 17% was produced (refer to Non-Patent Document 6). Furthermore, Aeromonas hydrophila was cultured using glucose and lauric acid as carbon sources, and the cell productivity of 50 g/L and polyester content of 50% were attained (refer to Non-Patent Document 7). However, Aeromonas hydrophila has pathogenicity to human (refer to Non-Patent Document 8), and thus cannot be said as a suitable species for the industrial production. Moreover, since expensive carbon sources are used in these culture productions, use of a cheap carbon source has also been asked for in view of the production cost.

Therefore, attempts for the production using a safe host and improvement of the productivity have been conducted. A polyhydroxyalkanoic acid (PHA) synthase gene was cloned from Aeromonas caviae (refer to Patent Document 5; and Non-Patent Document 9). As a result of producing P(3HB-co-3HH) using a transformant prepared by introducing this gene into Ralstonia eutropha (formerly Alcaligenes eutrophus), the cell productivity was 4 g/L and the polyester content was 30%. Moreover, as a result of culturing this transformant using a vegetable oil as a carbon source, the cell productivity of 4 g/L and polyester content of 80% were attained (refer to Non-Patent Document 10). Furthermore, researches on culture processes have also been conducted as can be seen in improvements of the cell productivity, polyester content and 3HH composition of up to 45 g/L, 62.5%, and 8.1%, respectively, by improving culture conditions (refer to Patent Document 6).

Moreover, Ralstonia eutropha capable of producing P(3HB-co-3HH) using fructose as a carbon source was also constructed, but this strain had low polyester productivity, and cannot be said to be suitable for the practical production (refer to Non-Patent Document 11).

A P(3HB-co-3HH)-producing strain using Escherichia coli as the host was also constructed. A strain prepared by introducing a PHA synthase gene of the genus Aeromonas, NADP-acetoacetyl Co-A reductase gene of Ralstonia eutropha, or the like, into Escherichia coli was constructed. As a result of culturing said Escherichia coli using dodecane as a carbon source for 40.8 hours, the cell productivity of 79 g/L, polyester content of 27.2%, and 3HH composition of 10.8% were obtained (refer to Non-Patent Document 12).

With the aim of improving the productivity of P(3HB-co-3HH) and controlling the 3HH composition, artificial modification of PHA synthase was carried out. Among PHA synthase mutants derived from Aeromonas caviae, a mutant enzyme in which serine is substituted for 149th amino acid asparagine and a mutant enzyme in which glycine is substituted for 171st aspartic acid showed improved PHA synthase activity in Escherichia coli and 3HH composition (refer to Non-Patent Document 13). Moreover, it was reported that a mutant enzyme in which isoleucine is substituted for 518th phenylalanine or a mutant enzyme in which glycine is substituted for 214th valine were improved in the PHA synthase activity in Escherichia coli and polyester content (refer to Non-Patent Document 14). However, since these mutants used particular Escherichia coli species as the host and still showed low polyester content, further improvements aiming at the industrial production making use of characteristics of these mutant enzymes have been required.

One of the most important subjects when PHAs are cultured in the industrial scale using a strain produced by applying recombinant DNA technologies is the stability of the transgene. For the gene transfer, a process comprising using a plasmid, a process comprising incorporation onto a host chromosome, and the like are used. However, a plasmid is known to be lost during proliferation and division of recombinant cells (refer to Patent Document 7). Therefore, cells from which a plasmid is lost lose PHA production ability, which leads to decrease of the commercial productivity. Conventionally, recombinant cells are generally cultured by selectively growing and retaining plasmid retention cells alone by adding antibiotics to a medium. However, problems such as a cost increase by the use of antibiotics, or influences to environment by residual antibiotics in a waste culture solution occur. Moreover, for stabilizing the plasmid, recombinant Escherichia coli prepared by introducing parB gene derived from R1 plasmid into P(3HB)-producing plasmid was produced (refer to Non-Patent Document 15). This Escherichia coli retained almost 100% of the plasmid even after culturing 110 to 120 generations. However, the plasmid still has the risk of being lost.

On the other hand, a gene incorporated onto a chromosome is thought to be stable. For this reason, a microorganism in which a gene involved with PHA synthesis was incorporated onto the chromosome was reported (refer to Patent Document 8, Non-Patent Document 16; and Non-Patent Document 17).

The Escherichia coli strain produced by randomly inserting a gene coding for a PHA biosynthesis enzyme onto the chromosome of Escherichia coli, as disclosed in Patent Document 8, produced P(3HB) at the level exceeding 85% of the dry cell weight. However, for making Escherichia coli produce P(3HB-co-3HH) having superior practical physical properties, it is required to make genes for supplying a substrate monomer coexist, or to supply expensive fatty acids, etc., to a medium. These requirements prevent attaining higher production efficiency. Furthermore, when the gene is randomly incorporated onto the chromosome, depending on the site to be incorporated, expression of the gene on that site or on the peripheral site thereof may be affected, and sufficient performances as a PHA-producing strain cannot be exhibited.

In the case of introducing an exogenous PHA synthase gene into a host in the form of plasmid or incorporating the same onto the chromosome, when a microbial species which inherently produces a PHA, such as the genus Ralstonia or the genus Pseudomonas, is used as the host, the strain which lost the inherent ability to produce a PHA has been used. Since such a strain is produced through mutation operations, the proliferation ability or biological activity is inferior to that of the parental strain (refer to Non-Patent Document 18; Non-Patent Document 19; Non-Patent Document 20; and the like), and it is hard to say that such a strain exhibits sufficient production ability as the PHA-producing strain.

On the other hand, in the report in which a PHA synthase gene derived from Chromatium vinosum is substituted for a PHA synthase gene on the chromosome of Ralstonia eutropha, P(3HB) was accumulated at 91% to the dry cell weight, which exceeded wild strains (Non-Patent Document 17). However, the cell productivity was as low as 1.8 g/L, and also the produced polyester was hard and brittle P(3HB), not P(3HB-co-3HH) with which a wide range of applications can be expected. Thus, for the commercial production of the polyester, there still have remained subjects to be solved.

Patent Document 1: Japanese Kokai Publication S57-150393
Patent Document 2: Japanese Kokai Publication S59-220192
Patent Document 3: Japanese Kokai Publication H05-93049
Patent Document 4: Japanese Kokai Publication H07-265065
Patent Document 5: Japanese Kokai Publication H10-108682
Patent Document 6: Japanese Kokai Publication 2001-340078
Patent Document 7: Japanese Kokai Publication S59-205983
Patent Document 8: U.S. Patent No. 6593116
Non-Patent Document 1: Madison et al., Microbiol. Mol. Biol. Rev., 63:21-53 (1999)
Non-Patent Document 2: Matsusaki et al., J. Bacteriol., 180:6459-6467 (1998)
Non-Patent Document 3: Matsusaki et al., Appl. Micrbiol. Biotechnol., 53:401-409 (2000)
Non-Patent Document 4: Langenbach et al., FEMS Microbiol. Lett., 150:303-309 (1997)
Non-Patent Document 5: Doi et al., Macromolecules, 28:4822-4828 (1995)
Non-Patent Document 6: Lee et al., Biotechnol. Bioeng., 67:240-244 (2000)
Non-Patent Document 7: Chen et al., Appl. Microbiol. Biotechnol., 57:50-55 (2001)
Non-Patent Document 8: "Safety Control Regulations on Pathogen, etc." issued by National Institute of Infectious Diseases; attached table 1, appended chart 1 (1999)
Non-Patent Document 9: Fukui et al., J. Bacteriol., 179:4821-4830 (1997)
Non-Patent Document 10: Fukui et al., Appl. Microbiol. Biotecnol., 49:333-336 (1998)
Non-Patent Document 11: Fukui et al., [0004] Environ. Microbiol., 68:2411-2419 (2002)
Non-Patent Document 14: Amara et al., Appl. Microbiol. Biotechnol., 59:477-482 (2002)
Non-Patent Document 15: Lee et al., J. Biotechnol., 32:203-211 (1994)
Non-Patent Document 16: Kranz et al., Appl. Environ. Microbiol., 63:3003-3009 (1997)
Non-Patent Document 17: York et al., J.Bacteriol., 183:4217-4226 (2001)
Non-Patent Document 18: Schlegel et al., Arch. Mikrobiol., 71:283-294 (1970)
Non-Patent Document 19: Schubert et al., J. Bacteriol., 170:5837-5847 (1988)
Non-Patent Document 20: Peoples et al., J. Biol. Chem., 264:15298-15303 (1989)

### SUMMARY OF THE INVENTION

As mentioned above, there still have been a lot of subjects for the commercial production of PHA.

The present invention has for its object to provide a recombinant microbial strain capable of accumulating a copolyester with which a broad range of applications can be expected stably at a high level in a culture process in the industrial scale, and to provide a production method of a copolyester using the same.

### DETAILED DESCRIPTION OF THE INVENTION

The present inventors made intensive investigations for solving the above subjects, and found that a polyester-producing microorganism prepared by substituting an exogenous polyhydroxyalkanoic acid synthase gene for a polyhydroxyalkanoic acid synthase gene on the chromosome can accumulate a PHA stably at a high level as described in claim 1.

For stabilizing the synthesis of PHA, it has been suggested effective to introduce a gene onto the chromosome. However, effects obtained when the PHA synthase gene of a wild strain is site-specifically substituted with another PHA synthase gene to accumulate copolyesters by disrupting the preexisting gene and expressing the transgene at the same time has not been known yet. Moreover, it could not be anticipated simply from the technologies which have been conventionally known.

As shown in Examples, a microbial strain prepared by substituting a polyhydroxyalkanoic acid synthase mutant gene derived from Aeromonas caviae for a polyhydroxyalkanoic acid synthase gene on the chromosome of a Ralstonia eutropha H16 strain produced P(3HB-co-3HH) having the cell production of 110.4 g/L and polyester content of 73.8 wt% by culturing for 48 hours. This productivity largely exceeded the productivity of P(3HB-co-3HH) which have so far been reported. Moreover, this strain did not require antibiotics or any other selective pressures through the whole culture processes, and substantially all the cells were found to have accumulated P(3HB-co-3HH) when the culture was completed.

That is, the present invention is described as follows.
[1] A microorganism of the genus Ralstonia, which originally has a polyhydroxyalkanoic acid synthase gene on its chromosome, in which at least a part of said polyhydroxyalkanoic acid synthase gene is substituted as defined in claim 1 with an exogenous polyhydroxyalkanoic acid synthase gene obtainable from Aeromonas caviae, and produces a copolyester constituted of two or more monomer units selected from the group consisting of 3-hydroxybutyric acid, 3-hydroxyhexanoic acid, 3-hydroxyheptanoic acid, 3-hydroxyoctanoic acid, 3-hydroxynonanoic acid, 3-hydroxydecanoic acid, 3-hydroxyundecanoic acid, 3-hydroxydodecanoic acid, 3-hydroxytridecanoic acid, 3-hydroxytetradecanoic acid, 3-hydroxypentadecanoic acid, and 3-hydroxyhexadecanoic acid.
[2] The microorganism according to [1], wherein the copolyester is constituted of monomer units of 3-hydroxybutyric acid and 3-hydroxyhexanoic acid.
[3] The microorganism according to [1] or [2], wherein the microorganism originally having a polyhydroxyalkanoic acid synthase gene on the chromosome is Ralstonia eutropha.
[4] The microorganism according to [3], wherein Ralstonia eutropha is a Ralstonia eutropha H16 strain.
[5] The microorganism according to [4], wherein the mutant is the one produced by at least one amino acid substitution (a) or (b):
   (a) substitution of serine for asparagine of the 149th amino acid;
   (b) substitution of glycine for aspartic acid of the 171st amino acid.
[6] The microorganism according to any one of [1] to [4], wherein the exogenous polyhydroxyalkanoic acid synthase gene codes for a mutant enzyme derived from Aeromonas caviae represented by the amino acid sequence shown under SEQ ID NO:9, which is prepared by substituting serine for asparagine of the 149th amino acid and substituting glycine for aspartic acid of the 171st amino acid.
[7] A production method of a polyester which comprises using the microorganism according to any one of [1] to [6].

The copolyester according to the present invention is a copolymer constituted of two or three or more monomer units selected from the group consisting of 3-hydroxybutyric acid, 3-hydroxyhexanoic acid, 3-hydroxyheptanoic acid, 3-hydroxyoctanoic acid, 3-hydroxynonanoic acid, 3-hydroxydecanoic acid, 3-hydroxyundecanoic acid, 3-hydroxydodecanoic acid, 3-hydroxytridecanoic acid, 3-hydroxytetradecanoic acid, 3-hydroxypentadecanoic acid and 3-hydroxyhexadecanoic acid, among polymers comprising, as a monomer unit, 3-hydroxyalkanoic acid represented by the following general formula: 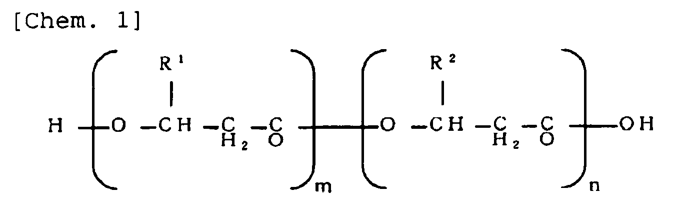 (wherein R¹ and R² represent an alkyl group having 1 or more carbon atom(s) and may be the same or different in the polymer; and m and n represent the numbers of monomer units of said polymer, and are 1 or more, respectively).

As the above copolyester, preferred is one constituted of the monomer units of 3-hydroxybutyric acid and 3-hydroxyhexanoic acid.

The microorganism to be used in the present invention is Ralstonia genus, more preferred is a Ralstonia eutropha, and still more preferred is a Ralstonia eutropha H16 strain.

The exogenous polyhydroxyalkanoic acid synthase gene to be used in the present invention is a polyhydroxyalkanoic acid synthase gene isolated from Aeromonas caviae (Non-Patent Document 9).

Moreover, it is also possible to use genes in which a part of the base sequence is modified for modifying the amino acid sequence within the range that the targeted enzymatic activity is not lost. For example, preferably used are a polyester synthase gene derived from Aeromonas caviae in which serine is substituted for 149th amino acid asparagine (N149S mutant gene) or a polyester synthase gene derived from Aeromonas caviae in which glycine is substituted for 171st amino acid aspartic acid (D171G mutant gene), both of them are described in Non-Patent Document 13, a polyester synthase gene derived from Aeromonas caviae represented by the amino acid sequence shown under SEQ ID NO:9 in which both of the above-mentioned amino acid substitution are combinedly carried out, or the like.

The above genes may have a promoter and/or ribosome binding site(s), but it is not necessarily required.

The aspect of substitution of the polyester synthase gene may be any form provided that the activity of the enzyme coded by the polyester synthase gene originally located on the chromosome (original gene) is lost and the exogenous polyester synthase gene (foreign gene) is expressed. After the substitution, all of the original genes may be lost from the chromosome, or a part thereof may occur, or they may exist in the divided form. As one aspect of substitution, when the foreign gene has a ribosome binding site and does not have a promoter, it is possible to select a mode in which the foreign gene is connected to the downstream of a promoter of the original gene. Moreover, as another aspect of substitution, when the foreign gene does not have a promoter and ribosome binding site, it is possible to select a mode in which the foreign gene is connected to the downstream of a ribosome binding site of the original gene. The mode of substitution may be a form in which the polyester synthase protein expressed after the substitution is a single protein coded by the foreign gene or a form in which the polyester synthase protein is a fusion protein with a protein coded by the original gene. The aspect of substitution of the polyester synthase gene used in the present invention is not restricted to those forms mentioned above, but preferably, the codons from the initiation codon to the terminal codon of the original gene are substituted with the codons from the initiation codon to the terminal codon of the foreign gene.

The process for site-specific substitution of a gene on the chromosome is well-known to the person skilled in the art. As a typical process, there are a process comprising using transposon and a homologous recombination mechanism (Ohman et al., J. Bacteriol., 162:1068-1074 (1985)), a process comprising as principles a site-specific incorporation caused by the homologous recombination mechanism and loss by homologous recombination of the second stage (Noti et al., Methods Enzymol., 154:197-217 (1987)), or the like. In addition, it is also possible to use a process comprising making sacB gene derived from Bacillus subtilis coexist with the foreign gene for easily isolating a microbial strain from which sacB gene is lost by homologous recombination of the second stage as a strain resistant to a sucrose-added medium (Schweizer, Mol. Microbiol., 6:1195-1204 (1992); Lenz et al., J. Bacteriol., 176:4385-4393 (1994)). However, the process is not particularly restricted provided that the gene on the chromosome can be substituted.
In the following, the process for substituting a PHA synthase gene derived from Aeromonas caviae (phaC_{Ac}) for a PHA synthase gene derived from Ralstonia eutropha (phaC_{Re}) is more specifically described.

First, a substitution fragment is produced. The substitution fragment has a form in which a coding sequence (CDS: initiation codon to termination codon are included) of phaC_{Ac} is connected to the upstream sequence just before CDS of phaC_{Re}, followed by the downstream sequence just after CDS of phaC_{Re}. That is, it is a DNA fragment of phaC_{Re} in which CDS alone is substituted with that of phaC_{Ac}. The upstream sequence and downstream sequence are homologous sequences necessary for causing homologous recombination with the gene on the chromosome. Generally, the longer its length, the higher the recombination frequency becomes, but the length can be optionally selected since only the homologous recombination is required.

To the substitution fragment, a gene which becomes a selective marker during the gene substitution can be added. As the gene to be a selective marker, for example, antibiotic-resistant genes such as a kanamycin-resistant gene, chloramphenicol-resistant gene, streptomycin-resistant gene and ampicillin-resistant gene, genes for complementing various nutrition requirements, and the like can be used. When Ralstonia eutropha is used, a kanamycin-resistant gene is suitably used.

In addition to those genes, it is also possible to add a gene for facilitating selection of a microbial strain from which a region containing the selective marker gene is lost by homologous recombination of the second stage. As such a gene, sacB gene derived from Bacillus subtilis can be mentioned (Schweizer, Mol. Microbiol., 6: 1195-1204 (1992)). A microbial strain in which this gene is expressed is known to be incapable of growing in a sucrose-containing medium, and by growing the strain in a sucrose-containing medium, it becomes easy to select the strain from which this gene is lost.

By connecting the substitution fragment constituted of these genes to a vector which does not replicate in a host microbial strain, a plasmid for gene substitution is produced. As such a vector which can be used in the genus Ralstonia, the genus Pseudomonas, or the like, there may be mentioned, for example, pUC vector, pBluescript vector or pBR322 vector, or vectors having the same replication origin as those, or the like. Moreover, it is also possible to make DNA sequences such as mob and oriT coexist, which enable conjugal transfer.

The plasmid DNA for gene substitution produced as mentioned above can be introduced into a microorganism such as Ralstonia eutropha by conventional methods such as an electroporation method or conjugal transfer method, and can be subjected to homologous recombination.

Next, a strain into which the plasmid DNA for gene substitution is inserted onto the chromosome by homologous recombination is selected. The selection can be conducted by a method based on the gene for selection which is made coexist in the plasmid DNA for gene substitution. When a kanamycin resistant gene is used, the strain can be selected from those grown in a kanamycin-containing medium.

In the next stage, the strain from which a region containing the selective marker gene is lost from the chromosome by the homologous recombination of the second stage is selected. Based on the gene for selection used in the insertion, a strain which becomes incapable of growing in a kanamycin-containing medium may be selected, for instance. However, when sacB gene coexists with a plasmid for gene substitution, it can be easily selected from strains growing in a sucrose-containing medium. For confirming whether the thus selected strain is one in which a gene is substituted as to be intended, conventional methods can be used such as a PCR method, southern hybridization method, and determination of the DNA base sequence. As mentioned above, a strain in which the PHA synthase gene (phaC_{Ac}) derived from Aeromonas caviae is substituted for the PHA synthase gene (phaC_{Re}) derived from Ralstonia eutropha can be obtained. As such a strain, there may be mentioned KNK-005 strain produced in the following Example.

In the practice of the present invention, copolyesters can be accumulated within the microorganisms by proliferating the microorganism of the invention under the existence of a carbon source. As the carbon source, sugar, oils and fats, or fatty acid can be used. As the nutrition source other than the carbon source, a nitrogen source, inorganic salts, and other organic nutrient sources can be optionally used.

As the sugar, there may be mentioned, for example, carbohydrates such as glucose and fructose. As the oils and fats, there may be mentioned oils and fats having 10 or more carbon atoms and containing a large amount of saturated and/or unsaturated fatty acids, for example, coconut oil, palm oil, palm kernel oil, and the like. As the fatty acid, there may be mentioned saturated and/or unsaturated fatty acids such as hexanoic acid, octanoic acid, decanoic acid, lauric acid, oleic acid, palmitic acid, linoleic acid, linolenic acid and myristic acid, or fatty acid derivatives, such as esters and salts of these fatty acids.

As the nitrogen source, there may be mentioned, for example, ammonia, ammonium salts such as ammonium chloride, ammonium sulfate and ammonium phosphate, peptone, meat extract, yeast extract, and the like.

As the inorganic salts, there may be mentioned, for example, potassium dihydrogenphosphate, dipotassium hydrogenphosphate, magnesium phosphate, magnesium sulfate, sodium chloride, and the like.

As the other organic nutrient sources, there may be mentioned, for example, amino acids such as glycine, alanine, serine, threonine and proline; vitamins such as vitamin B1, vitamin B12 and vitamin C; and the like.

The culture temperature may be any temperatures as long as the cells can grow, and preferred as this temperature is 20 to 40°C. The culture period is not particularly restricted, and may be about 1 to 10 days.

Polyesters accumulated in the obtained culture cells can be recovered by conventional methods.

Polyesters can be recovered by the following methods, for example. After completion of the culture, the cells are separated from the culture fluid using a centrifugal machine, etc., washed with distilled water, methanol, etc., and then dried. From these dried cells, polyesters are extracted using an organic solvent such as chloroform. From the polyester-containing organic solvent, cell components are removed by filtration, etc., and a poor solvent such as methanol or hexane is added to the filtrate to precipitate polyesters. Then, polyesters can be recovered by carrying out filtration or centrifugation to remove the supernatant fluid and then carrying out drying.

As a simple method for confirming production of polyesters, a staining method using Nile red can be used. That is, whether polyesters are produced can be confirmed by a method comprising adding Nile red to an agar medium in which recombinant cells grow, culturing the recombinant cells for 1 to 7 days, and observing whether the recombinant cells turn red or not.

According to the present invention, a recombinant microbial strain capable of stably producing a polyhydroxyalkanoic acid (PHA) at a high production rate in an industrial fermentation process can be provided, and a highly-pure polyhydroxyalkanoic acid (PHA) can be easily produced in a large amount at a low cost.

### BRIEF DESCRIPTION OF THE DRAWINGS

Fig. 1 is a scheme view showing the constitution of the plasmid for gene substitution pBlue-phaC_{Ac}::N149S/D171G-KmSAC constructed in Example 1.
Fig. 2 is a scheme view showing the procedure of gene substitution carried out in Example 2.

### BEST MODE FOR CARRYING OUT THE INVENTION

The following examples illustrate the present invention, however, these examples are by no means limitative of the scope of the present invention.

### (Example 1) Construction of a plasmid for gene substitution

A PCR reaction was carried out using cells of a Ralstonia eutropha H16 strain as a supply source of a template DNA and using primers as shown under SEQ ID NO:1 and NO:2, and a DNA fragment containing a structural gene of polyhydroxyalkanoic acid synthase gene (phaC_{Re}) was obtained. The PCR reaction conditions were as follows: (1) at 94°C for 2 minutes, (2) at 94°C for 30 seconds, (3) at 45°C for 30 seconds, (4) at 72°C for 3 minutes, 25 cycles of (2) to (4), and (5) at 72°C for 5 minutes. As a polymerase, TaKaRa LA Taq (product of TAKARA BIO INC.) was used. The DNA fragment obtained by PCR was cleaved with the restriction enzyme BamHI, and subcloned into the vector pBluescriptIIKS (-) (product of TOYOBO CO., LTD.) at the site which had already been cleaved with the same enzyme (pBlue-phaC_{Re}).

N149S/D171G mutant, which is a polyester synthase mutant gene derived from Aeromonas caviae, was produced as follows. First, pBluescriptIIKS (-) (product of TOYOBO CO., LTD.) was treated with PstI, blunted using DNA Blunting Kit (product of TAKARA BIO INC.), subjected to ligation, and the PstI site was deleted. Then, this plasmid was treated with XhoI, blunted using DNA Blunting Kit (product of TAKARA BIO INC.), subjected to ligation, and the plasmid pBlue-New from which the XhoI site was deleted was obtained. To an EcoRI site of this plasmid, a d13 fragment cleaved from pJRD215-EE32d13 (Patent Document 5) with the same enzyme was cloned (pBlue-d13). Then, a plasmid derived from a clone E2-50 distributed from Institute of Physical and Chemical Research (Non-Patent Document 13) was used as a template, amplification was carried out by PCR using a set of primers shown under SEQ ID NO:3 and NO:4 and a set of primers shown under SEQ ID NO:5 and NO:6 to obtain two fragments. The conditions were as follows: (1) at 94°C for 2 minutes, (2) at 94°C for 30 seconds, (3) at 55°C for 30 seconds, (4) at 72°C for 2 minutes, 25 cycles of (2) to (4), and (5) at 72°C for 5 minutes. The amplified two fragments were mixed in equivalent moles, and a PCR reaction was carried out again to couple said two fragments. The conditions were as follows: (1) at 96°C for 5 minutes, (2) at 95°C for 2 minutes, (3) at 72°C for 1 minute, and 12 cycles of (2) to (3). As a polymerase, Pyrobest polymerase (product of TAKARA BIO INC.) was used. A DNA fragment with the objective size was extracted from an agarose electrophoresis gel, treated with PstI and XhoI, and then substituted with pBlue-d13, which had already been treated with PstI and XhoI, in order to accomplish cloning (pBlue-N149S/D171G). The base sequence was determined using DNA sequencer 310 Genetic Analyzer manufactured by PERKIN ELMER APPLIED BIOSYSTEMS, and it was confirmed as a mutant gene of the PHA synthase gene in which serine is substituted for 149th amino acid asparagine, and glycine is substituted for 171st aspartic acid. The amino acid sequence is shown under SEQ ID:9.

Using pBlue-N149S/D171G as a template, a PCR reaction was carried out using primers shown under SEQ ID NO:7 and NO:8, a structural gene DNA of N149S/D171G mutant was amplified. The PCR conditions were as follows: (1) at 94°C for 2 minutes, (2) at 94°C for 30 seconds, (3) at 45°C for 30 seconds, (4) at 72°C for 2 minutes, 25 cycles of (2) to (4), and (5) at 72°C for 5 minutes. As a polymerase, TaKaRa LA Taq (product of TAKARA BIO INC.) was used. Then, pBlue-phaC_{Re} was treated with the restriction enzymes SbfI and Csp45I, and the above-amplified DNA fragment, which had already been treated with SbfI and Csp45I, was substituted with phaC_{Re} structural gene, in order to accomplish cloning (pBlue-phac_{Re}::N149S/D171G) .

Next, plasmid pJRD215 (ATCC37533) was treated with the restriction enzymes XhoI and DraI to isolate a DNA fragment of about 1.3 kb containing a kanamycin-resistant gene, and the resulting fragment was blunted using DNA Blunting Kit (product of TAKARA BIO INC.). This fragment was inserted into pBlue-phaC_{Re}::N149S/D171G at the site which had already been cleaved with the restriction enzyme SalI and then blunted in the same manner as above (pBlue-phaC_{Re}::N149S/D171G-Km).

Subsequently, plasmid pMT5071 (Tsuda, GENE, 207:33-41 (1998)) was treated with the restriction enzyme NotI to isolate a DNA fragment of about 8 kb containing sacB gene. The fragment was inserted into pBlue-phaC_{Re}::N149S/D171G-Km at the site which had already been cleaved with NotI, and the plasmid for gene substitution pBlue-phaC_{Re}::N149S/D171G-KmSAC was produced.

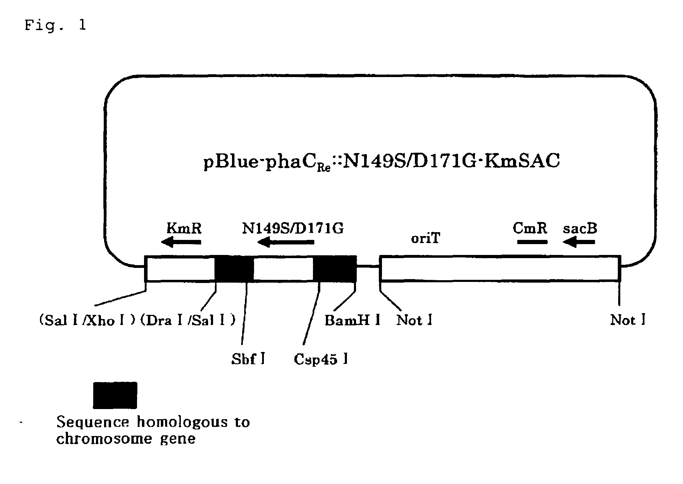

A scheme view showing the constitution of the plasmid for gene substitution pBlue-phaC_{Re}::N149S/D171G-KmSAC constructed in this Example is shown in Fig. 1.

### (Example 2) Construction of a gene-substituted strain

An Escherichia coli S17-1 strain (ATCC47005) was transformed with the plasmid for gene substitution pBlue-phaC_{Re}::N149S/D171G-KmSAC, and subjected to mixed culture with a Ralstonia eutropha H16 strain on Nutrient Agar medium (product of DIFCO Laboratories) to carry out conjugal transfer. A strain grown on Simmons agar medium containing 250 mg/L of kanamycin (sodium citrate 2 g/L, sodium chloride 5 g/L, magnesium sulfate-heptahydrate 0.2 g/L, ammonium dihydrogenphosphate 1 g/L, dipotassium hydrogenphosphate 1 g/L, agar 15 g/L, pH 6.8) was selected, and a strain in which the plasmid is incorporated onto the chromosome of the Ralstonia eutropha H16 strain was obtained (homologous recombination of the first stage). This strain was cultured on Nutrient Broth medium (product of DIFCO Laboratories) through two generations, and diluted and applied on Nutrient Agar medium containing 15% of sucrose. Grown strains were selected to obtain the strains from which a region containing a selective marker is lost (homologous recombination of the second stage). Moreover, by analysis using PCR, a strain in which phaC_{Re} gene was substituted with N149S/D171G mutant gene was isolated. This gene-substituted strain was named KNK-005 strain, and the base sequence was determined using DNA sequencer 310 Genetic Analyzer manufactured by PERKIN ELMER APPLIED BIOSYSTEMS. It was confirmed as a strain in which the codons from the initiation codon to terminal codon of phaC_{Re} gene on the chromosome were substituted with the codons from the initiation codon to terminal codon of N149S/D171G mutant gene.

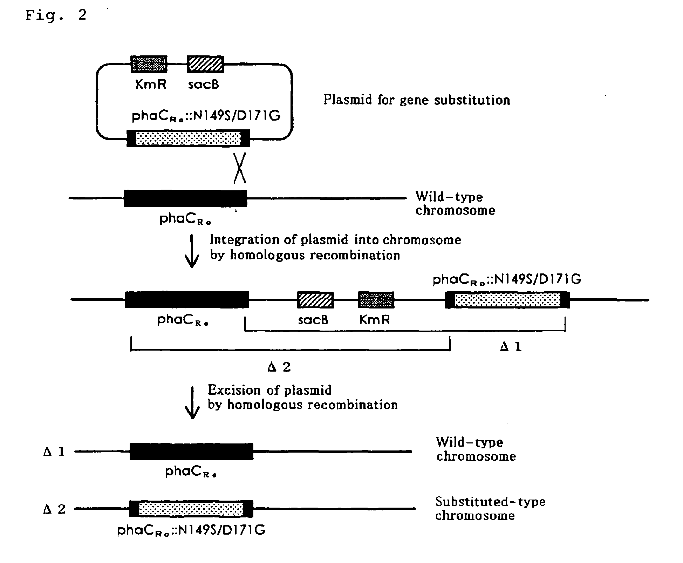

The procedure of gene substitution carried out in this Example is schematically shown in Fig. 2. By the homologous recombination of the first stage, the plasmid for gene substitution is integrated into the chromosome, and by the homologous recombination of the second stage, a part corresponding to the plasmid again forms a ring to be excised from the chromosome. During the homologous recombination of the second stage, depending on the site where homologous recombination occurs, the plasmid is excised together with the same foreign gene as the original one (phaC_{Re}::N149S/D171G in Fig.2) (Δ1) or together with the original gene (phaC_{Re} in Fig.2) (Δ2). The KNK-005 strain of the present invention is obtained by homologous recombination of the second stage carried out in the location of Δ2.

### (Example 3) Production and purification of polyester

The composition of a seed culture medium was set as follows: 1 w/v% of Meat-extract, 1 w/v% of Bacto-Trypton, 0.2 w/v% of Yeast-extract, 0.9 w/v% of Na₂PO₄·12H₂O, 0.15 w/v% of KH₂PO₄, and pH 6.8.

The composition of a preculture medium was set as follows: 1.1 w/v% of Na₂PO_{4·}12H₂O, 0.19 w/v% of KH₂PO₄, 1.29 w/v% of (NH₄)₂SO₄, 0.1 w/v% of MgSO₄·7H₂O, 2.5 w/v% of palm W oleic oil, and 0.5 v/v% of solution containing trace amount of a metal salt (prepared by dissolving 1.6 w/v% of FeCl₃· 6H₂O, 1 w/v% of CaCl₂ · 2H₂O, 0.02 w/v% of CoCl₂·6H₂O, 0.016 w/v% of CuSO₄· 5H₂O, and 0.012 w/v% of NiCl₂· 6H₂O in 0.1 N hydrochloric acid).

The composition of a polyester-producing medium was set as follows: 0.385 w/v% of Na₂PP₄·12H₂O, 0.067 w/v% of KH₂PO₄, 0.291 w/v% of (NH₄)₂SO₄, 0.1 w/v% of MgSO₄·7H₂O, 0.5 v/v% of solution containing trace amount of a metal salt (prepared by dissolving 1.6 w/v % of FeCl₃· 6H₂O, 1 w/v % of CaCl₂ · 2H₂O, 0.02 w/v% of CoCl₂ - 6H₂O, 0.016 w/v% of CuSO₄ · 5H₂O, and 0.012 w/v% of NiCl₂.6H₂O in 0.1 N hydrochloric acid), and 0.05 w/v% BIOSPUREX 200K (defoaming agent: product of Cognis Japan Ltd.). As a carbon source, palm kernel oil olein, which is a low-melting point fraction prepared by fractionating palm kernel oil, was used as a single carbon source, and fed at the specific supply rate of substrate of 0.08 to 0.1 (g oils and fats) x (g net weight of dry cells)⁻¹ x (h)⁻¹ through the whole culture.

A glycerol stock (50 µl) of KNK-005 strain was inoculated into the seed culture medium (10 ml) and cultured for 24 hours, and 1.0 v/v% of the resultant was inoculated in a 3L jar fermentor (product of B. E. Marubishi, Co., Ltd.; MDL-300 type) containing 1.8 L of the preculture medium. The operation conditions were as follows: culture temperature 30°C, stirring rate 500 rpm, and aeration amount 1.8 L/min. The culture was carried out for 28 hours while controlling the pH between 6.7 and 6.8. For the pH control, a 7% aqueous solution of ammonium hydroxide was used.

The polyester production culture was carried out by inoculating 5.0 v/v% of the preculture seed to a 10 L jar fermentor (product of B. E. Marubishi, Co., Ltd.; MDL-1000 type) containing 6 L of the production medium. The operation conditions were as follows: culture temperature 28°C, stirring rate 400 rpm, aeration amount 3.6 L/min, and the pH was controlled between 6.7 and 6.8. For the pH control, a 7% aqueous solution of ammonium hydroxide was used. The culture was carried out for about 48 hours. After completion of the culture, the cells were recovered by centrifugation, washed with methanol, and then freeze-dried. The weight of the dry cells was determined, and was found to be 110.4 g/L.

To about 1 g of the obtained dry cells, 100 ml of chloroform was added and the mixture was stirred at room temperature through a day and night to extract polyesters within the cells. After filtrating the cell residues, the polyesters were concentrated to about 30 ml in the total volume using an evaporator, gradually added with about 90 ml of hexane, and left for 1 hour while slowly stirring. The precipitated polyesters were filtrated, and dried in vacuum at 50°C for 3 hours. The weight of the dry polyesters was determined and the polyester content within the cells was calculated. Consequently, the polyester content obtained by KNK-005 strain was as high as 73.8 (wt%) in 48 hours.

### (Example 4) Evaluation of stability of polyester-producing ability

The bacterial culture liquid obtained in Example 3 at the completion of the polyester production culture was diluted and inoculated to Nutrient Agar medium. The colonies which have grown were inoculated to a Nile red-containing medium (9 g of disodium hydrogenphosphate· dodecahydrate, 1.5 g of potassium dihydrogenphosphate, 0.05 g of ammonium chloride, 0.02 g of magnesium sulfate· heptahydrate, 0.5 g of fructose, 0.25 ppm of cobalt chloride·hexahydrate, 16 ppm of iron (III) chloride-hexahydrate, 10.3 ppm of calcium chloride·dihydrate, 0.12 ppm of nickel chloride·heptahydrate, 0.16 ppm of copper sulfate·pentahydrate, 0.5 mg of Nile red, and 15 g/l L of agar). After culturing the colonies at 30°C for 3 days, those turning red can be judged as they produce polyesters. 100 colonies were evaluated, and all of the colonies were found to have the polyester-producing ability.

### INDUSTRIAL APPLICABILITY

According to the present invention, a recombination microbial strain capable of stably producing a polyhydroxyalkanoic acid (PHA) at a high production rate in an industrial fermentation process can be provided, and a highly-pure polyhydroxyalkanoic acid (PHA) can be easily produced in a large amount at a low cost.

## Claims

1. A microorganism
which originally has a polyhydroxyalkanoic acid synthase gene on its chromosome, in which at least a part of said polyhydroxyalkanoic acid synthase gene is substituted on the chromosome with an exogenous polyhydroxyalkanoic acid synthase gene so that the activity of an enzyme coded by the polyhydroxyalkanoic acid synthase gene on the chromosome is lost and the exogenous polyhydroxyalkanoic acid synthase gene is expressed, and
which produces a copolyester constituted of two or more monomer units selected from the group consisting of 3-hydroxybutyric acid, 3-hydroxyhexanoic acid, 3-hydroxyheptanoic acid, 3-hydroxyoctanoic acid, 3-hydroxynonanoic acid, 3-hydroxydecanoic acid, 3-hydroxyundecanoic acid, 3-hydroxydodecanoic acid, 3-hydroxytridecanoic acid, 3-hydroxytetradecanoic acid, 3-hydroxypentadecanoic acid, and 3-hydroxyhexadecanoic acid,
wherein the microorganism is genus Ralstonia, and
wherein the exogenous polyhydroxyalkanoic acid synthase gene codes for an enzyme obtainable from Aeromonas caviae.

2. The microorganism according to Claim 1,
wherein the copolyester is constituted of monomer units of 3-hydroxybutyric acid and 3-hydroxyhexanoic acid.

3. The microorganism according to Claim 1 or 2,
wherein the microorganism originally having a polyhydroxyalkanoic acid synthase gene on the chromosome is Ralstonia eutropha.

4. The microorganism according to Claim 3,
wherein Ralstonia eutropha is a Ralstonia eutropha H16 strain.

5. The microorganism according to any one of Claims 1 to 4,
wherein the exogenous polyhydroxyalkanoic acid synthase gene is an exogenous polyhydroxyalkanoic acid synthase gene derived from Aeromonas caviae with at least one amino acid substitution (a) or (b):
(a) substitution of serine for asparagine of the 149th amino acid;
(b) substitution of glycine for aspartic acid of the 171 st amino acid.

6. The microorganism according to any one of Claims 1 to 5,
wherein the exogenous polyhydroxyalkanoic acid synthase gene codes for a mutant enzyme derived from Aeromonas caviae represented by the amino acid sequence shown under SEQ ID NO:9, which is prepared by substituting serine for asparagine of the 149th amino acid and substituting glycine for aspartic acid of the 171 st amino acid.

7. A production method of a polyester
which comprises using the microorganism according to any one of Claims 1 to 6.

## Patentansprüche

1. Mikroorganismus
welcher ursprünglich ein Polyhydroxyalkansäuresynthase-Gen auf dessen Chromosom hat, in dem zumindest ein Teil des Polyhydroxyalkansäuresynthase-Gens auf dem Chromosom mit einem exogenen Polyhydroxyalkansäuresynthase-Gen substituiert ist, sodass die Aktivität eines Enzyms, das durch das Polyhydroxyalkansäuresynthase-Gen auf dem Chromosom kodiert ist, verloren geht, und das exogene Polyhydroxyalkansäuresynthase-Gen exprimiert wird, und
welcher einen Copolyester, der aus zwei oder mehr Monomereinheiten gebildet ist, die ausgewählt sind aus der Gruppe, bestehend aus 3-Hydroxybutansäure, 3-Hydroxyhexansäure, 3-Hydroxyheptansäure, 3-Hydroxyoctansäure, 3-Hydroxynonansäure, 3₋Hydr,oxydecansäure, 3-Hydroxyundecansäure, 3-Hydroxydodecansäure, 3-Hydroxytridecansäure, 3-Hydroxytetradecansäure, 3-Hydroxypentadecansäure und 3-Hydroxyhexadecansäure, produziert,
wobei der Mikroorganismus von der Gattung Ralstonia ist, und
wobei das exogene Polyhydroxyalkansäuresynthase-Gen für ein Enzym kodiert, das von Aeromonas caviae erhältlich ist.

2. Mikrooganismus gemäß Anspruch 1, wobei der Copolyester aus Monomereinheiten von 3-Hydroxybutansäure und 3-Hydroxyhexansäure gebildet ist.

3. Mikroorganismus gemäß Anspruch oder 2, wobei der Mirkoorganismus, der ursprünglich ein Polyhydroxyalkansäuresynthase-Gen auf dem Chromosom hat, Ralstonia eutropha ist.

4. Mikroorganismus gemäß Anspruch 3, wobei Ralstonia eutropha ein Ralstonia eutropha H₁₆-Stamm ist.

5. Mikroorganismus gemäß einem der Ansprüchen 1 bis 4, wobei das exogene Polyhydroxyalkansäuresynthase-Gen ein exogenes Polyhydroxyalkansäuresynthase-Gen, abgeleitet von Aeromonas caviae, mit zumindest einer Aminosäuresubstitution (a) oder (b) ist:
(a) Substitution von Serin für Asparagin der 149. Aminosäure;
(b) Substitution von Glycin für Asparaginsäure der 171. Aminosäure.

6. Mikroorganismus gemäß einem der Ansprüchen 1 bis 5, wobei das exogene Polyhydroxyalkansäuresynthase-Gen für ein Mutantenzym, abgeleitet von Aeromonas caviae, kodiert, das durch die Aminosäuresequenz, die unter SEQ ID NO: 9 gezeigt ist, dargestellt ist, welches durch Substitution von Serin für Asparagin der 149. Aminosäure und Substitution von Glycin für Asparaginsäure der 171. Aminosäure hergestellt wird.

7. Herstellungsverfahren eines Polyesters, welches die Verwendung des Mikroorganismus gemäß einem der Ansprüchen bis 6 umfasst.

## Revendications

1. Micro-organisme
dont le chromosome comporte à l'origine un gène d'acide polyhydroxyalcanoïque synthase, dans lequel au moins une partie dudit gène d'acide polyhydroxyalcanoïque synthase sur le chromosome est substitué par un gène d'acide polyhydroxyalcanoïque synthase exogène de manière à perdre l'activité d'une enzyme codée par le gène d'acide polyhydroxyalcanoïque synthase sur le chromosome et à exprimer le gène d'acide polyhydroxyalcanoïque synthase exogène et
qui produit un copolyester constitué de deux unités monomères ou plus, choisies parmi le groupe formé par l'acide 3-hydroxybutyrique, l'acide 3-hydroxyhexanoïque, l'acide 3-hydroxyheptanoïque, l'acide 3-hydroxyoctanoïque, l'acide 3-hydroxynonanoïque, l'acide 3-hydroxydécanoïque, l'acide 3-hydroxyundécanoïque, l'acide 3-hydroxydodécanoïque, l'acide 3-hydroxytridécanoïque, l'acide 3-hydroxytétradécanoïque, l'acide 3-hydroxypentadécanoïque et l'acide 3-hydroxyhexadécanoïque, le micro-organisme étant du genre Ralstonia et
le gène d'acide polyhydroxyalcanoïque synthase exogène codant pour une enzyme qu'il est possible d'obtenir de Aeromonas caviae.

2. Micro-organisme selon la revendication 1, dans lequel le copolyester est constitué d'unités monomères d'acide 3-hydroxybutyrique et d'acide 3-hydroxyhexanoïque.

3. Micro-organisme selon la revendication 1 ou 2, dans lequel le micro-organisme dont le chromosome comporte à l'origine un gène d'acide polyhydroxyalcanoïque synthase est Ralstonia eutropha.

4. Micro-organisme selon la revendication 3, dans lequel Ralstonia eutropha est une souche H 16 de Ralstonia eutropha.

5. Micro-organisme selon l'une des revendications 1 à 4, dans lequel le gène d'acide polyhydroxyalcanoïque synthase exogène est un gène d'acide polyhydroxyalcanoïque synthase exogène dérivé d'Aeromonas caviae comportant au moins une substitution (a) ou (b) d'acide aminé :
(a) l'asparagine du 149^{ème} acide aminé est substituée par la sérine,
(b) l'acide aspartique du 171^{ème} acide aminé est substitué par la glycine.

6. Micro-organisme selon l'une des revendications 1 à 5, dans lequel le gène d'acide polyhydroxyalcanoïque synthase exogène code pour une enzyme mutante dérivée d'Aeromonas caviae représentée par la séquence d'acide aminé montrée à l'identificateur de séquence n° 9 qui est préparée en substituant la serine à l'asparagine du 149^{ème} acide aminé et en substituant la glycine à l'acide aspartique du 171^{éme} acide aminé.

7. Procédé pour produire un polyester, lequel comprend l'utilisation du micro-organisme selon l'une des revendications 1 à 6.


### SEQUENCE LISTING

<110> KANEKA Corporation
   <110> Meredian, Inc.
<120> MICROORGANISM WITH REPLACED GENE AND PROCESS FOR PRODUCING POLYESTER USING THE SAME
<130> P26652
<140> PCT/JP2006/314498
   <141> 2006-07-21
<160> 9
<170> PatentIn version 3.1
<210> 1
   <211> 27
   <212> DNA
   <213> Artificial
<220>
   <223> Primer
<400> 1
   gtcggatccc gggcaagtac cttgccg 27
<210> 2
   <211> 27
   <212> DNA
   <213> Artificial
<220>
   <223> primer
<400> 2
   gacggatcca tggtgtcgac cagcttg 27
<210> 3
   <211> 26
   <212> DNA
   <213> Artificial
<220>
   <223> primer
<400> 3
   aggttctggc cgccggactc cagggt 26
<210> 4
   <211> 32
   <212> DNA
   <213> Artificial
<220>
   <223> primer
<400> 4
   ggagagcata tgagccaacc atcttatggc cc 32
<210> 5
   <211> 26
   <212> DNA
   <213> Artificial
<220>
   <223> primer
<400> 5
   accctggagt ccggcggcca gaacct 26
<210> 6
   <211> 55
   <212> DNA
   <213> Artificial
<220>
   <223> primer
<400> 6
   atggatcctg cagttagtgg tggtggtggt ggtgtgcggc gtcctcctct gttgg 55
<210> 7
   <211> 63
   <212> DNA
   <213> Artificial
<220>
   <223> primer
<400> 7
   gccggttcga atagtgacgg cagagagaca atcaaatcat gagccaacca tcttatggcc 60
   cgc 63
<210> 8
   <211> 83
   <212> DNA
   <213> Artificial
<220>
   <223> primer
<400> 8
   gggaacctgc aggcctgccg gcgccgtgca tgacgcacgc cggcactcat gcaagcgtca 60
   tgcggcgtcc tcctctgttg ggc 83
<210> 9
   <211> 594
   <212> PRT
   <213> Aeromonas caviae
<220>
   <223> variant of polyhydroxyalkanoate synthetic enzyme
<400> 9
   Met Ser Gln Pro Ser Tyr Gly Pro Leu Phe Glu Ala Leu Ala His Tyr
   1               5                   10                  15
   Asn Asp Lys Leu Leu Ala Met Ala Lys Ala Gln Thr Glu Arg Thr Ala
               20                  25                  30
   Gln Ala Leu Leu Gln Thr Asn Leu Asp Asp Leu Gly Gln Val Leu Glu
           35                  40                  45
   Gln Gly Ser Gln Gln Pro Trp Gln Leu Ile Gln Ala Gln Met Asn Trp
       50                  55                  60
   Trp Gln Asp Gln Leu Lys Leu Met Gln His Thr Leu Leu Lys Ser Ala
   65                  70                  75                  80
   Gly Gln Pro Ser Glu Pro Val Ile Thr Pro Glu Arg Ser Asp Arg Arg
                   85                  90                  95
   Phe Lys Ala Glu Ala Trp Ser Glu Gln Pro Ile Tyr Asp Tyr Leu Lys
   100 105 110
   Gln Ser Tyr Leu Leu Thr Ala Arg His Leu Leu Ala Ser Val Asp Ala
   115 120 125
   Leu Glu Gly Val Pro Gln Lys Ser Arg Glu Arg Leu Arg Phe Phe Thr
   130 135 140
   Arg Gln Tyr Val Ser Ala Met Ala Pro Ser Asn Phe Leu Ala Thr Asn
   145 150 155 160
   Pro Glu Leu Leu Lys Leu Thr Leu Glu Ser Gly Gly Gln Asn Leu Val
   165 170 175
   Arg Gly Leu Ala Leu Leu Ala Glu Asp Leu Glu Arg Ser Ala Asp Gln
   180 185 190
   Leu Asn Ile Arg Leu Thr Asp Glu Ser Ala Phe Glu Leu Gly Arg Asp
   195 200 205
   Leu Ala Leu Thr Pro Gly Arg Val Val Gln Arg Thr Glu Leu Tyr Glu
   210 215 220
   Leu Ile Gln Tyr Ser Pro Thr Thr Glu Thr Val Gly Lys Thr Pro Val
   225 230 235 240
   Leu Ile Val Pro Pro Phe Ile Asn Lys Tyr Tyr Ile Met Asp Met Arg
   245 250 255
   Pro Gln Asn Ser Leu Val Ala Trp Leu Val Ala Gln Gly Gln Thr Val
   260 265 270
   Phe Met Ile Ser Trp Arg Asn Pro Gly Val Ala Gln Ala Gln Ile Asp
   275 280 285
   Leu Asp Asp Tyr Val Val Asp Gly Val Ile Ala Ala Leu Asp Gly Val
   290 295 300
   Glu Ala Ala Thr Gly Glu Arg Glu Val His Gly Ile Gly Tyr Cys Ile
   305 310 315 320
   Gly Gly Thr Ala Leu Ser Leu Ala Met Gly Trp Leu Ala Ala Arg Arg
   325 330 335
   Gln Lys Gln Arg Val Arg Thr Ala Thr Leu Phe Thr Thr Leu Leu Asp
   340 345 350
   Phe Ser Gln Pro Gly Glu Leu Gly Ile Phe Ile His Glu Pro Ile Ile
   355 360 365
   Ala Ala Leu Glu Ala Gln Asn Glu Ala Lys Gly Ile Met Asp Gly Arg
   370 375 380
   Gln Leu Ala Val Ser Phe Ser Leu Leu Arg Glu Asn Ser Leu Tyr Trp
   385 390 395 400
   Asn Tyr Tyr Ile Asp Ser Tyr Leu Lys Gly Gln Ser Pro Val Ala Phe
   405 410 415
   Asp Leu Leu His Trp Asn Ser Asp Ser Thr Asn Val Ala Gly Lys Thr
   420 425 430
   His Asn Ser Leu Leu Arg Arg Leu Tyr Leu Glu Asn Gln Leu Val Lys
   435 440 445
   Gly Glu Leu Lys Ile Arg Asn Thr Arg Ile Asp Leu Gly Lys Val Lys
   450 455 460
   Thr Pro Val Leu Leu Val Ser Ala Val Asp Asp His Ile Ala Leu Trp
   465 470 475 480
   Gln Gly Thr Trp Gln Gly Met Lys Leu Phe Gly Gly Glu Gln Arg Phe
   485 490 495
   Leu Leu Ala Glu Ser Gly His Ile Ala Gly Ile Ile Asn Pro Pro Ala
   500 505 510
   Ala Asn Lys Tyr Gly Phe Trp His Asn Gly Ala Glu Ala Glu Ser Pro
   515 520 525
   Glu Ser Trp Leu Ala Gly Ala Thr His Gln Gly Gly Ser Trp Trp Pro
   530 535 540
   Glu Met Met Gly Phe Ile Gln Asn Arg Asp Glu Gly Ser Glu Pro Val
   545 550 555 560
   Pro Ala Arg Val Pro Glu Glu Gly Leu Ala Pro Ala Pro Gly His Tyr
   565 570 575
   Val Lys Val Arg Leu Asn Pro Val Phe Ala Cys Pro Thr Glu Glu Asp
   580 585 590
   Ala Ala